(19) Europäisches Patentamt
European Patent Office
Office européen des brevets

(11) Numéro de publication: **0 189 694**
**B1**

(12) **FASCICULE DE BREVET EUROPÉEN**

(45) Date de publication du fascicule du brevet:
**10.08.88**

(51) Int. Cl.⁴: **C 07 C 125/065,** C 07 C 127/00,
C 07 C 118/00

(21) Numéro de dépôt: **85402511.1**

(22) Date de dépôt: **17.12.85**

(54) Procédé de synthèse d'isocyanates et de dérivés d'isocyanates.

(30) Priorité: **28.12.84 FR 8419969**

(43) Date de publication de la demande:
**06.08.86 Bulletin 86/32**

(45) Mention de la délivrance du brevet:
**10.08.88 Bulletin 88/32**

(84) Etats contractants désignés:
**BE CH DE GB IT LI NL**

(56) Documents cité:
**US-A-3 914 311**

**BULL. OF THE CHEMICAL SOCIETY OF JAPAN, vol.
53, no. 12, décembre 1980, pages 3691-3695, The
Chemical Society of Japan, Tokyo, JP; K. TAKAGI
et al.: "The in Situ-generated nickel(0)-catalyzed
reaction of aryl halides with potassium iodide and
zinc powder"**

(73) Titulaire: **SOCIETE NATIONALE DES POUDRES ET
EXPLOSIFS, 12, quai Henri IV, F-75181 Paris
Cédex 04 (FR)**

(72) Inventeur: **Tkatchenko, Igor, 25 D rue Lassagne,
F-69300 Caluire (FR)**
Inventeur: **Jaouhari, Rabih, 11 B rue du Bois-
Perrin Appartement 208, F-35000 Rennes (FR)**
Inventeur: **Bonnet, Michel, 62 rue de la
Garonnette, F-31000 Toulouse (FR)**
Inventeur: **Dawkins, Gordon, 37 New Mains Road,
Kirkliston EH29 9AW Scotland (GB)**
Inventeur: **Lecolier, Serge, 3 allée des Cartelines,
F-91510 Janville sur Juine (FR)**

**Description**

L'invention concerne un nouveu procédé de sythèse d'isocyanates et de dérivés d'isocyanates à partir d'halogènures organiques et de cyanates métalliques, selon la séquence réactionnelle générale:

$$RX + NCOM \rightarrow MX + RNCO \overset{ZN}{\rightarrow} RNHCOZ \tag{1}$$

dans laguelle R représente un groupement organigue, X un halogène, M un métal et ZH un composé organigue à hydrogène mobile, par exemple un alcool, ou une amine primaire ou secondaire.

Les dérivés d'isocyanates particulièrement concernés sont les carbamates et les urées. Ces dérivés et les isocyanates sont notamment utilisés soit comme agents de synthèse fine pour la production de pesticides et de médicaments, soit comme monomères ou co-monomères dans la préparation de nombreux composés macromoléculaires comme les polyuréthannes dont les emplois sont très variés.

La synthèse d'isocyanates et de dérivés d'isocyanates selon la séquence réactionnelle (1) est connue.

S. OZAKI (Chem. Rev. 1972, 72, 457) et plus récemment les articles de K FINDEISEN, K. KÖNIG, R. SUNDERMAN (Houben-Weyl Methoden der Organischen Chemie Vol. E 4, 1984, pp. 738-784) et J. IPPEN (ibid., pp. 784-802) résument les domaines d'application de la séquence réactionnelle (1). En particulier la réaction a lieu dans des conditions relativement douces lorsque des halogénures d'alkyle sont employés, les bromures étant plus réactifs que les chlorures. Les halogénures benzyliques et allyliques sont également réactifs. Ces articles par contre ne décrivent pas la synthèse d'arylisocyanates et de vinylisocyanates selon cette méthode.

Plusieurs brevets décrivent différentes améliorations apportées à la synthèse de carbamates, d'alkyl-isocyanates, d'allyl-isocyanates et de benzyl-isocyanates. L'amélioration la plus couramment décrite consiste en la solubilisation de l'ion cyanate en milieu organique, généralement aprotique dipolaire.

Dans ce but, la demande de brevet japonais 7 584 528 décrit l'emploi d'éthers couronnes et de cyanates de tétraalkylammonium, et EP 88 218 le cyanate de bis (pyridine) zinc qui résulte de l'interaction entre cyanates alcalins, chlorure de zinc et pyridine.

- US 3 558 684 décrit la préparation d'alkenyl isocyanates par réaction d'un halogénure d'alkényle avec un cyanate de métal alcalin en présence d'un catalyseur constitué de cuivre métallique et d'un halogénure cuivreux.

- US 4 130 577 décrit la préparation d'isocyanates benzyliques par réaction d'un halogénure benzylique avec un cyanate de métal alcalin en présence d'un catalyseur constitué d'un métal (V, Mn, Co, Zn, Pd, Sn) et de sels de ces métaux.

- FR 2 464 249 décrit un procédé de synthèse de l'isocyanate de méthyle par réaction de $CH_3Cl$ avec NCOK en présence de KI ou de KBr.

- Les demandes de brevet japonais 7 584 528 et 7 584 529 mentionnent explicitement l'obtention de l'isocyanate de phényle par réaction du chlorobenzène avec le cyanate de potassium en condition de transfert de phase.

La synthèse d'isocyanates hétérocycliques ou aromatiques ou vinyliques par réaction d'un hétérocycle halogéné ou d'un composé halogéné aromatique ou vinylique avec un cyanate métallique en milieu exclusivement organique n'est pas, à la connaissance de la Demanderesse décrite.

Il est bien connu que la réactivité vis à vis d'agents nucléophiles, des liaisons carbone sp2-halogène telles que rencontrées dans les halogénures vinyliques et aréniques est beaucoup plus faible que celle des liaisons carbone sp2-halogène (CK. Ingold, "Structure and Mechanisms in Organic Chemistry", 2ème ed., Cornell University press, Ithaca, 1969, p. 559). La présente invention a notamment pour but de remédier à ce manque de réactivité.

Le procédé selon l'invention de synthèse d'isocyanates et de dérivés d'isocyanates est caractérisé en ce que la réaction, en milieu organique, entre l'halogénure organique et le cyanate métallique a lieu en présence d'un catalyseur constitué par un complexe du nickel avec au moins un ligand organique, complexe dans lequel le nickel est au degré d'oxydation zéro.

Les dérivés d'isocyanates particulièrement concernés ont la formule générale RNHCOZ dans laquelle R représente un groupement organique et Z un groupement choisi dans le groupe constitué par les groupements alcoxy et aminés ($NH_2$, non, mono ou di-substitué). On les obtient en faisant réagir l'isocyanate formé par réaction entre l'halogénure organique et le cyanate métallique avec un composé de formule ZH, Z ayant la signification précitée.

Lorsque ZH est un composé hydroxylé, on obtient un carbamate et lorsque ZH est une amine primaire ou secondaire on obtient une urée.

A titre d'exemple illustratifs et non limitatifs, ZH peut être l'éthanol, le méthanol, l'isopropanol, le t-butanol, l'éthyl-2 hexanol, le cyclohexanol, l'éthylène glycol, un phénol, un crésol, un naphtol, la diméthylamine, la diéthylamine, la butylamine, l'aniline.

De façon préférée, le composé de formule ZH est introduit dans le milieu réactionnel an une fois au début de la réaction entre l'halogénure organique et le cyanate métallique.

Selon une autre variante particulièrement préférée, la composé de formule ZH est ajouté dans le milieu réactionnel en au moins une introduction pendant la réaction entra l'halogénure organique et le cyanate métallique. Par exemple, le composé ZH peut être ajouté en 2 introductions, en début de réaction puis aux deux tiers du temps de réaction global.

2

Le rapport molaire composé ZH/halogénure organique de départ peut être quelconque mais est de préférence voisin de 1. De façon particulièrement préférée, ce rapport est légèrement supérieur à 1.

Le procédé selon l'invention permet par conséquent d'obtenir d'une façon simple et économique de nombreux isocyanates et leurs dérivés comportant différentes fonctions insensibles aux conditions réactionnelles, sans faire intervenir le phosgène, composé particulièrement toxique, y compris et il faut bien apprécier l'importance de ce fait, les isocyanates hétérocyliques ou aromatiques ou vinyliques et leurs dérivés.

De façon préférée, l'halogénure organique est choisi dans le groupe constitué par:

- les halogénures vinyliques, acycliques ou cycliques, éventuellement substitués par au moins un groupement alkyle ou aryle. On peut citer par exemple le bromure de vinyle, le bromo-1 propène-1, le bromo-1 phényl-2 éthylène.

- les halogénures aromtiques simples ou condensés, éventuellement substitués par au moins un groupement alkyle, vinyle, aryle, halogène, alcoxy. On peut citer par exemple le chloro, bromo ou iodobenzène, le parabromoanisole, le parabromotoluène, le parabrochlorobenzène, le parabromofluorobenzène, les chloro ou bromonaphtalènes,

- les composés hétérocycliques halogénés comme les bromopyridines et le bromo-3 furanne;

- les halogénures allyliques, éventuellement substitués par au moins un groupement alkyle ou aryle, tels que le bromure d'allyle, le chlorure de méthyl-2 allyle, le bromure de cinnamyle;

- les halogénures aliphatiques linéaires ou cycliques ou ramifiés, tels que le chloro, bromo ou iodométhane, le chloro-1 butane, le dichloro 1-4 butane, le bromocyclohexane;

- les halogénures benzyliques, éventuellement substitués par au moins un groupement alkyle ou aryle tels que le chlorure ou bromure de benzyle et les chlorure ou bromure de cumyle.

De façon particulièrement préférée, l'halogénure organique est choisi dans le groupe constitué par les halogénures vinyliques précités et les halogénures aromatiques précités.

D'une manière générale, on obtint de meilleurs rendements avec les iodures et bromures qu'avec les chlorures. L'ordre de réactivité observé est le suivant:

$$F << Cl < Br \cong I$$

Une caractéristique particulière de l'invention est qu'on peut accroître la réactivité des chlorures, et par conséquent le rendement de la réaction, en ajoutant au milieu réactionnel un bromure ou iodure alcalin ou d'onium, ce qui revient à un échange chlore/brome (ou iode) dans le chlorure organique. L'addition d'une fraction molaire de l'ordre de 10 % suffit généralement pour accroître de manière substantielle la conversion du chlorure organique.

De façon préférée, le cyanate métallique utilisé est un cyanate alcalin ou alcalino-terreux. Les cyanates de sodium ou de potassium, commerciaux, ont un intérêt pratique évident. On utilisera plus particulièrement le cyanate de sodium, plus soluble que le cyanate de potassium. Bien évidemment, tous les cyanates métalliques conviennent et permettent de réaliser l'invention.

Le rapport molaire NCOM/RX peut être quelconque mais est choisi de préférence légèrement supérieur à 1; un rapport compris entre 1,15 et 1,35 est particulièrement préféré.

Le procédé selon l'invention est caractérisé en ce que la réaction entre l'halogénure organique et le cyanate métallique a lieu en présence d'un catalyseur constitué par un complexe du nickel avec au moins un ligand organique.

De façon préférée le (ou les) ligand organique est phosphoré mais les ligands arséniés par exemple ou exclusivement hydrocarbonés comme les ligands cyclooctadiène ou cyclododécatriène conviennent également.

Lorsque le (ou les) ligand est phosphoré, il peut être monodentate ou multidentate. Les phosphines basiques et les phosphines bidentates sont particulièrement préférées. Par phosphines bidentates, on entend les composés phosphorés de structure générale $R_1R_2P-(CH_2)_n-PR_3R_4$ dans laquelle les substituants $R_1$, $R_2$, $R_3$, $R_4$ sont des groupements alkyle ou aryle. Parmi les phosphines basiques on peut citer notamment les tributyl, trihexyl et trioctylphosphines, la tricyclohexylphosphine, la diméthylphénylphosphine et la méthyldiphénylphosphine.

Les complexes $Ni(PPh_3)_4$, $Ni(PPh_3)_3$, $Ni(PBu_3)_4$ et $Ni(PCy_3)_2$, Ph signifiant phényle, Bu butyle, Cy cyclohexyle, sont préférés.

Parmi les phosphines bidentates, on peut citer les bis (diphénylphosphino) polyméthylène et notamment les bis (diphénylphosphino) éthane et butane (soit en abrégé respectivement dppe et dppb) qui sont préférés. On constate l'ordre d'activité suivant des complexes

$$Ni [Ph_2P (CH_2)_n PPh_2]_2 : n = 1 < 2 > 3 < 4$$

Dans ce cas, le rapport nickel/phosphore correspond à celui des complexes préformés soit 1/4, mais il sera, de préférence, choisi égal à 1/2, comme par exemple dans les complexes $Ni(Ph_2P (CH_2)_nPPh_2)$ formés in situ.

La préparation de tels complexes phosphorés peut être réalisée selon la méthode décrite dans Inorg. Synth. 1977, 17, 121 mais est de façon préférée réalisée avantageusement in situ par l'addition d'un équivalent de diphosphine ou de 2 équivalents de monophosphine à des complexes zérovalents du nickel tels que le bis (cyclooctadiène) nickel (soit en abrégé Ni(cod)$_2$), ou le (cyclododécatriène-1, 5, 9) nickel eux-mêmes préparés selon la méthode décrite dans Inorg. Synth. 1974, 15, 5.

On peut également préparer et utiliser un complexe zérovalent du nickel ayant un ligand phosphine basique et un ligand phosphine bidentate comme le complexe Ni (dppe) (PPh$_3$)$_2$.

Lorsque le (ou les) ligand est arsénié, on préferera une arsine tertiaire comme par exemple la triphénylarsine.

A titre d'exemple les catalyseurs Ni (As Ph$_3$)$_4$ et Ni (As Ph$_3$)$_2$ formés _in situ_ conviennent parfaitement.

Il peut être avantageusement préparé _in situ_ par l'addition de 2x moles de triphénylarsine à x moles d'un complexe zérovalent du nickel tel que le bis (cyclooctadiène) nickel ou le (cyclododécatriène-1, 5, 9) nickel.

Lorsque le (ou les) ligand organique n'est ni phosphoré ni arsénié, il peut, à titre d'exemple non limitatif, être un hydrocarbure cyclique comme le cyclooctadiène ou le cyclododécatriène. Les complexes zérovalents du nickel tels que le bis (cyclooctadiène) nickel le (cyclododécatriène-1, 5, 9) nickel conviennent parfaitement pour réaliser l'invention.

Les complexes selon l'invention sont généralement employés à des concentrations comprises entre 10$^{-2}$ et 10$^{-3}$ mole/litra.

Il est par aillaurs important de rappeler et souligner que seuls les complexas, dans lesquels le nickel est à l'état d'oxydation zéro, conviennent pour réaliser l'invention.

Dans le cas de la préparation d'isocyanates, carbamates et urées à partir d'halogénures aromatiques, on améliore le rendement en ajoutant dans le milieu réactionnel de faibles quantités d'un acide de Lewis.

Un tel ajout, dans d'autres cas, et notamment lors de la préparation de carbamates vinyliques ou hétérocycliques ou benzyliques ou d'alkyle, entraîne au contraire une perte d'efficacité du système catalytique (chute du rendement, très importante dans certains cas).

Parmi les acides de Lewis utilisables, on peut par exemple citer l'éthérate de trifluorure de bore, le chlorure d'aluminium, le chlorure de zinc, le chlorure ferrique et le chlorure stanneux.

On les utilise à des concentrations faibles, comparables à celles des complexes du nickel précités. Le rapport molaire entre l'acide de Lewis et le complexe est généralement compris entre 0,5 et 2. Un rapport supérieur à 2 entraîne une baisse du rendement.

La présence d'un solvant organique dans lequel sont solubles l'halogénure organique, le catalyseur et l'acide de Lewis lorsqu'il est présent, et dans lequel la solubilité du cyanate métallique utilisé n'est pas nulle, est indispensable.

De manière générale, bien que des solvants non polaires tels que les hydrocarbures saturés ou aromatiques puissent convenir dans la mesure où la réaction est conduite en présence des halogénures d'onium déjà mentionnés, on préférera un solvant aprotique dipolaire, éventuellement en mélange avec un solvant hydrocarboné.

De ce fait on choisira le solvant parmi les éthers, esters, nitriles, sulfoxydes ou les amides de point d'ébullition relativement élevé. A titre d'exemple, le diglyme, le carbonate de propylène, le N,N-diméthylformamide (DMF), le N,N-diméthylacétamide (DMAC), la N-méthyl pyrrolidone (NMP), éventuellement en mélange avec la ligroïne, le toluène ou un xylène conviennent bien.

La présence d'eau a une influence néfaste sur le cours de la réaction, ce qui impose l'emploi de solvants anhydres pour la mise en oeuvre de la réaction. Une caractéristique particulière de l'invention consiste à éliminer les traces d'eau, avant l'addition du catalyseur, par un entraînement azéotropique, au moyen de toluène ou de xylène, de l'eau du mélange réactionnel.

La température du milieu réactionnel peut être comprise entra +20°C et 160°C. La température optimale dépend notamment de l'halogénure organique. Elle se situe entre 140°C at 155°C dans le cas des halogénures aromatiques ou vinyliques ainsi que pour les hétérocycles halogénés. Elle peut être beaucoup plus basse dans le cas des halogénures allyliques.

Lorsque les halogénures de départ sont volatils, la réaction peut être effectuée en tube de Schlenk ou de Carius ou en autoclave sous la pression autogène du substrat tout en maintenant une agitation suffisante pour assurer un bon contact au sein du milieu réactionnel entre le cyanate métallique et la solution organique.

La réaction est poursuivie pendant le temps nécessaire à l'obtention d'un taux de conversion satisfaisant de l'halogénure organique. Ce temps de réaction dépend de la nature de l'halogénure organique et peut varier de 1 à 30 heures.

Les exemples suivants, nullement limitatifs, illustrent l'invention.

## Example 1

Préparation du phénylcarbamate d'éthyle à partir du bromobenzène
Addition de l'éthanol on une fois.

Dann un tube de Schlenk de 20 ml muni d'un barreau aimanté at purgé à l'argon, on introduit successivement:

- 0,375 g (5,5 mmol) de cyanate de sodium sec,
- 0,085 g (0,1 mmol) du complexe Ni (Ph$_2$-P-CH$_2$CH$_2$-P-Ph$_2$)$_2$

préparé selon la méthode décrite dans Inorg. Synth. 1977, 17, 121,

- 2,5 ml de DMF redistillé sur alumine,
- 0,679 g (4,3 mmol) de bromobenzène,
- 270 µl d'éthanol (4,6 mmol).

On agite et chauffe dans un bain d'huile thermostaté à 155°C le mélange pendant 27 h, puis on le ramène à la température ambiante. Par chromatographie en phase gazeuse (CPG), on détermine un taux de conversion de 68 % du bromobenzène et un rendement de 27,5 % en phénylcarbamate d'éthyle.

**Exemple 2**

Préparation du phényl carbamate d'éthyle à partir du bromobenzène >
Addition de l'éthanol en deux fois.

Dans un tube de Schlenk de 20 ml muni d'un barreau aimanté et purgé à l'argon, on introduit successivement:
- 0,344 g (5,3 mmol) de cyanate de sodium sec,
- 0,079 g (0,093 mmol) du complexe Ni (Ph$_2$P-CH$_2$-CH$_2$-PPh$_2$)$_2$
- 2,4 ml de DMF redistillé sur alumine,
- 0,663 g (4,2 mmol) de bromobenzène,
- 140 µl d'éthanol (2,4 mmol)

On agite et chauffe dans un bain d'huile thermostaté à 155°C le mélange pendant 16h. On refroidit rapidement le tube de Schlenk puis on rajoute sous argon 140 µl (2,4 mmol) d'éthanol. On agite et chauffe à 155°C le mélange pendant 11h, puis on le ramèna à la température ambiante.
Par CPG, on détermine un taux de conversion du bromobenzène de 72 % et un rendement en phénylcarmabate d'éthyle de 35 %.

**Exemple 3**

Préparation du phénylcarbamate d'éthyle à partir du bromobenzène
Isolement du produit.

Dans un tube de Schlenk de 100 ml muni d'un barreau aimanté et purgé à l'argon, on introduit successivement:
- 1,96 g (30 mmol) de cyanate de sodium sec,
- un mélange de 0,152 g (0,55 mmol) de bis (cyclooctadiène-1,5) nickel préparé selon la méthode décrite dans Inorg. Synth. 1974, 15,5 et de 0,222 g (0,55 mmol) de bis (diphénylphosphino)-1,2 éthane,
- 0,058 g (0,3 mmol) de chlorure stannaux sec,
- 16,5 ml de DMAC redistillé sur alumine,
- 3,69 g (23,4 mmol) de bromobenzène,
- 750 µl (12,8 mmol) d'éthanol.
On agita et chauffe dans un bain d'huile thermostaté à 155°C le mélange pendant 16 h. On refroidit rapidement le tube de Schlenk puis on rajoute sous argon 750 µl (12,8 mmol) d'éthanol. On agite et chauffe à 155°C le mélange pendant 11 h, puis on le ramène à la température abiante. On filtre le mélange réactionnel puis on distille le solvant. On reprend le résidu par de l'éther (3 x 10 ml). On évapore l'éther de la phase éthérée puis on distilla la phénylcarbamate d'éthyle du résidu. (Eb : 100°C sous 5 mm Hg).
Le rendement obtenu est 33,5 %. La produit a été identifié par ses spectres IR et RMN:
Spectre IR (dans CH$_2$Cl$_2$) bandes caractéristiques à 3460 et 1730 cm$^{-1}$
Spectre RMN[1]H (dans CCl$_4$)
δ = 1,25 ppm (3H, t, J$_{HH}$ = 7 Hz)
δ = 4,1 ppm (2H, q, J$_{HH}$ = 7 Hz)
δ = 6,8 - 7,5 ppm (6H, massif)

**Exemple 4**

Préparation du phénylcarbamate d'éthyle
Amélioration apportée par la pratique d'un entraînement azétropique.

Dans un ballon bicol de 250 ml muni d'un barraau aimanté et purgé à l'argon, on introduit successivement:
- 2,36 g (36,4 mmol) de cyanate de sodium sec,
- 4,45 g (28,2 mmol) de bromobenzène,
- 0,067 g (0,36 mmol) de chlorure stanneux,
- 20 ml de DMAC redistillé sur alumine,
- 60 ml de toluène sec.

On chauffe le mélange au reflux du toluène puis on distille celui-ci. Après refroidissement du ballon on ajoute un mélange de 0,182 g (0,655 mmol) de bis (cyclooctadiène-1,5) nickel et de 0,260 g (0,655 mmol) de bis (diphénylphosphino)-1,2 éthane puis 0,90 ml (15,5 mmol) d'éthanol.

On agite et chauffe dans un bain d'huile thermostaté à 155°C le mélange pendant 16 h. On refroidit rapidement le ballon puis on rajoute sous argon 0,90 ml (15,5 mmol) d'éthanol. On agite et chauffe à 155°C le mélange pendant 11 h puis on le ramène à température ambiante. On traite ensuite le mélange comme dans l'exemple 3. Le rendement en phénylcarbamante d'éthyle isolé est de 42 %.

### Exemples 5-32

Préparation du phénylcarbamate d'éthyle à partir du bromobenzène

Influence de divers paramètres réactionnels (nature et concentration des réactifs et catalyseurs, nature du solvant, mode d'introduction de l'éthanol).

Le tableau I rassemble les données et résultats de différents essais effectués selon les conditions "standard" explicitées par les exemples 1, 2 (addition de l'éthanol en 2 fois), 3 et 4 (formation d'un complexe de nickel zérovalent in situ et addition d'un acide de Lewis).

Ce tableau met notamment en évidence l'intérêt de l'addition fractionnée de l'éthanol et de la présence d'un acide de Lewis dans une plage relativement limitée de concentration. Concernant ce dernier point, on peut constater qu'au delà d'une certaine limite supérieure de concentration, la présence de l'acide de Lewis entraîne au contraire une chute considérable du rendement.

L'exemple 30 a été réalisé avec du cyanate de baryum. Ce composé n'étant pas commercialisé, il a été préparé par réaction, dans l'éther, de l'acide isocyanique avec le carbonate de baryum.

La réaction entre le bromobenzène, le cyanate de baryum at l'éthanol a été suivie par chromatographie sur couche mince (CCM). Cette technique a permis de mettre en évidence la formation de phénylcarbamate d'éthyle.

### Exemples 33 et 34

Préparation du phénylcarbamate d'éthyle à partir du chlorobenzène.

On prépare une solution de catalyseur par dissolution sous atmosphère d'argon de 0,9 g de $Ni(cod)_2$ et 1,3 g de diphénylphosphine éthane dans 100 ml de diméthylacétamide.

Dans un tube de Schlenk de 500 $cm^3$, on introduit 23,65 g (364 mmoles) de NaOCN, 0,6825 g (3,6 mmoles) de $SnCl_2$ et 300 ml de toluène.

On évapore par distillation sous pression atmosphérique la moitié du toluène, puis on distille le reste du toluène sous pression réduite pour obtenir un résidu sec.

On introduit ensuite, toujours sous atmosphère d'argon, 0,9 g de $Ni(cod)_2$, 1,3 g de diphénylphosphine éthane, 28,7 ml (282 moles) de chlorobenzène, 9 ml (155 mmoles) d'éthanol at 100 ml de la solution de catalysaur préparée ci-dessus.

La quantité de catalyseur ainsi ajoutée est de 1,8 g (6,55 mmoles) de $Ni(cod)_2$ avec 2,6 g de (diphénylphosphino)éthane.

Le tube de Schlenk est maintenu 16 heures à 155°C. Après refroidissement, on rajoute 9 ml (155 mmoles) d'éthanol et on réchauffe 10 heures à 155°C.

Après refroidissement, on évapore le diméthylacétamide et on reprend le résidu avec 150 ml d'éther. Après filtration et évaporation de l'éther, le phénylcarbamate d'éthyle est distillé sous pression réduite.

Le produit isolé a une pureté de 92 % en phénylcarbamate d'éthyle. Le rendement de la réaction est de 37,5 %.

On réalise un essai semblable à l'essai précédent, à la différence que la quantité de catalyseur ajoutée est de 0,3174 g de $Ni (cod)_2$. On isole 13,5 g de phénylcarbamate d'éthyle à 90 % de pureté. Le rendement obtenu en carbamate pur est de 26 %.

6

**Exemples 35 et 36**

Préparation de la N-phényl N',N'-dibutylurée à partir du bromobenzène et de la dibutylamine.

Exemple 35:

Dans un tube de Schlenk de 20 ml muni d'un barreau aimanté et purgé à l'argon, on introduit successivement:

- 0,36 g (5,5 mmol) de cyanate de sodium sec,
- un mélange de 0,028 g (0,1 mmol) de bis (cyclooctadiène-1,5), nickel et de 0,04 g (0,1 mmol) de bis (diphénylphosphino)éthane,
- 0,100 g (0,055 mmol) de chlorure stanneux sec,
- 3 ml de DMAC redistillé sur alumine,
- 0,68 g (4,3 mmol) de bromobenzène,
- 0,60 g (4,7 mmol) de dibutylamine

On agite et chauffe le mélange dans un bain d'huile thermostaté à 155°C pendant 27 h. On refroidit le mélange réactionnel jusqu'à la température ambiante.

On filtre le mélange réactionnel. On distille le solvant puis la N-phényl N',N'-dibutylurée (120°C sous 0,1 mm Hg). Le taux de conversion du bromobenzène est de 50 %. Le rendement en N-phényl N',N'-dibutylurée ainsi isolée est de 35 %.

La N-phényl N', N'-dibutylurée obtenue, identifiée par ses spectres RMN et IR, a un point de fusion de 82°C.
Spectre IR (dans $CH_2Cl_2$) bandes caractéristiques à 3490 cm$^{-1}$ et 1690 cm$^{-1}$.
Spectre RMN$^1$H (dans $CDCl_3$)
$\delta$ = 6,97 - 7,53 ppm (6H, m)
$\delta$ = 3,07 3,7 ppm (4H, m)
$\delta$ = 0,72 - 1,72 ppm (14H, m)

Exemple 36:

Même mode opératoire que pour l'exemple 33 mais sans la présence de chlorure stanneux.
On isole la N-phényl N', N'-dibutylurée avec un rendement de 28,5 %.

**Exemple 37**

Préparation de la N-styryl N', N'-diéthylurée à partir du bromostyrène et de la diéthylamine.

La même procédure générale que pour l'exemple 34 a été suivie, dans les conditions particulières précisées au tableau II.

On distille la N-styryl N',N'-diéthylurée à 95-100°C sous 0,1 mm Hg. Le rendement en N-styryl N',N'-diéthylurée ainsi isolée ast de 48 %. La pureté, déterminée par CPG, est supérieure à 93 %. La N-styryl N',N'-diéthylurée obtenue a été identifiée par ses spectres IR et RMN:
- IR (dans le Nujol): bandes caractéristiques à 3340 cm$^{-1}$, 2950 cm$^{-1}$, 1670 cm$^{-1}$ et 1630 cm$^{-1}$.
- RMN$^1$H (dans $CDCl_3$); déplacements chimiques en ppm par rapport au TMS:
  * 6,75 - 7,36 : multiplet correspondant aux 5 protons du cycle phényle
  * 5,07 - 6,65 : système AB, $J_{AB}$ = 14 Hz (2 protons éthyléniques + Proton N-H)
  * 3,21 : quadruplet correspondant aux 4 protons $CH_2$ des 2 chaînes éthyle $J_{HH}$ = 7 Hz
  * 1,18 : triplet correspondant aux 6 protons $CH_3$ des 2 chaînes éthyle $J_{HH}$ = 7 Hz.

**Exemple 38**

Préparation de la N-butyl N', N'-diéthylurée à partir de chlorure de butyle et de la diéthylamine.

La même procédure générale que pour l'exemple 33 a été suivie, dans les conditions particulières précisées au tableau II.

On distille la N-butyl N',N'-diéthylurée à 60°C sous 0,1 mm Hg. Le rendement en N-butyl N',N'-diéthylurée ainsi isolée est de 35 %. La N-butyl N',N'-diéthylurée a été identifiée par CPG (comparaison avec produit étalon) et par son spectre IR (bandes caractéristiques à 3340 cm$^{-1}$ et 1670 cm$^{-1}$).

7

**Exemples 39 à 64**

Préparation de différents arylcarbamates d'éthyle à partir d'halogénures aromatiques.

Le tableau III regroupe les essais mettant en oeuvre des mono ou poly halogénures aromatiques simples ou condensés, mono ou polysubstitués, éventuellement en présence d'un sel d'onium.

Dans le cas des dihalogénures, les rendements indiqués sont ceux en monocarbamate. Dans le cas des dihalogénures bromés dont l'autre atome d'halogène est différent du brome, les rendements indiqués sont ceux en monocarbamate obtenu par substition du brome.

Les résultats montrent la généralité de la réaction et la nécessité d'employer un catalyseur selon l'invention pour observer un rendement notable.

**Exemples 65-73**

Préparation du phényl-2 vinylcarbamate d'éthyle à partir du bromo-1 phényl 2 éthylène (mélange des isomères cis et trans).

Le mode opératoire général est celui décrit dans les exemples 1 à 3.

Les conditions expérimentales particulières à chaque essai sont précisées dans le tableau IV ainsi que les résultats obtenus. Ces résultats montrent l'influence particulièrement néfaste pour le rendement de la réaction de la présence d'un acide de Lewis, pourtant utilisé à la même concentration que celle permettant une hausse de ce même rendement dans le cas des halogénures aromatiques.

**Exemple 74**

Préparation du phényl-2 vinylcarbamate d'éthyle à partir du bromo-1 phényl-2 éthylène.

Isolement du produit de la réaction.

Dans un tube de Schlenk de 20 ml muni d'un barreau aimanté et purgé à l'argon, on introduit successivement:

- 0,278 g (4,28 mmol) de cyanate de sodium sec,
- 0,021 g (0,025 mmol) de complexe Ni (Ph$_2$P CH$_2$ CH$_2$ PPh$_2$)$_2$
- 2,65 ml de DMF redistillé sur alumine,
- 0,656 g (3,6 mmol) de bromo-1 phényl-2 éthylène,
- 210 µl (4,6 mmol) d'éthanol.

On agite et chauffe dans un bain d'huile thermostaté à 143°C le mélange pendant 26 h. On ramène ensuite le mélange à la température ambiante puis on distille le solvant sous pression réduits.

On purifie le résidu sur une colonne d'alumine basique (20 x 2 cm, 50 g Al$_2$O$_3$). L'élution avec des mélanges toluène-acétate d'éthyle conduit à l'obtention de 0,502 g (2,6 mmol) de trans phényl-2 vinylcarbamate d'éthyle, soit un rendement de 73 %. Le produit a été identifié par spectrométries IR et RMN:
- spectre IR (dans le nujol) bandes caractéristiques à 3340, 1700 et 1660 cm$^{-1}$
- spectre RMN$^1$H (dans CDCl$_3$)
$\delta$ = 1,3 ppm (3H, t, J$_{HH}$ = 7 Hz)
$\delta$ = 4,3 ppm (2H, q, J$_{HH}$ = 7 Hz)
$\delta$ = 6,0 ppm (2H, AB, J$_{AB}$ = 14 Hz)
$\delta$ = 7,3 ppm (6H, massif).

**Exemples 75 à 82**

Préparation de différents vinylcarbamates d'éthyle à partir d'halogénures vinyliques.

Le tableau V rassemble les essais mettant en oeuvre divers halogénures vinyliques simples ou substitués.

Les résultats montrent la généralité de la réaction.

L'essai 82 est effectué dans un autoclave en acier inoxydable muni d'un pot en verre et d'un barreau aimanté sous la pression autogène du dérivé vinylique, à la température désignée.

## Exemples 83 à 88

Préparation de carbamates d'éthyle à partir d'hétérocycles halogénés.

Le tableau VI rassemble les conditions opératoires et résultats d'essais mettant en oeuvre différents composés hétérocycliques halogénés.

La présence d'un acide de Lewis entraîne une baisse du rendement.

## Exemples 89 à 98

Préparation d'allylcarbamates, de benzylcarbamates et d'alkylcarbamates d'éthyle.

Le tableau VII rassemble les conditions opératoires at résultats d'essais mettant an oeuvra différants halogénures allyllques, benzyliques ou aliphatiques.

Les résultats montrant l'amélioration apportée par l'amploi d'un catalysaur selon l'invention et l'inopportunité de mettre an oeuvre la réaction an présence d'un acide de Lewis (baissa de rendeau).

## Exemples 99 et 100

Préparation du phénylisocyanate à partir de cyanate de sodium et de bromobenzène.

Exemple 99:
Dans un tube de Schlenk de 100 ml muni d'un barreau aimanté et purgé à l'argon on introduit successivement:

- 1,44 g (22,2 mmol) de cyanate de sodium sec,
- un mélange de 0,111 g (0,4 mmol) de bis (cyclooctadiène-1,5) nickel et de 0,16 g (0,4 mmol) de bis (diphénylphosphino)-1,2 éthane (soit 0,4 mmol du complexe Ni (dppe))
- 0,042 g (0,22 mmol) de chlorure stanneux sec,
- 10 ml de NMP redistillé sur alumine,
- 2,71 g (17,2 mmol) de bromobenzène.

On agite et chauffe dans un bain d'huile thermostaté à 155°C le mélange pendant 27 h.
Après refroidissement, on filtre le mélange. On ajoute 35 ml d'éther au filtrat puis on récupère la phase éthérée. On filtre la phase éthérée puis on évapore l'éther. On distille le produit obtenu et on récupère la tête de distillation qui contient du bromobenzène de départ n'ayant pas réagi (point d'ébullition 156°C), l'isocyanate de phényle formé (point d'ébullition 162°C) et des traces de NMP (point d'ébullition 210°C). Ces produits sont séparés par passage, sous argon, sur colonne de silice préalablement chauffée 10 min à 50°C.
L'élution avec des mélanges toluène-acétate d'éthyle permet d'isoler le phénylisocyanate.
Le phénylisocyanate obtenu a été identifié par son indice de réfraction et ses spectres IR et RMN:

$n_D^{20} = 1,5360$

Spectre IR: $\nu$ NCO = 2250 cm$^{-1}$
Spectre RMN$^1$H (dans CCl$_4$): multiplet à 6,8 - 7,5 ppm (par rapport au TMS) correspondant aux 5 protons aromatiques.

Exemple 100:
Même mode opératoire que pour l'exemple 99 mais sans la présence de chlorure stanneux.
Le phénylisocyanate obtenu a été isolé et identifié de la même façon que pour l'exemple 99.

**Exemple 101**

Préparation du bromo-4 phénylisocyanate à partir de cyanate de sodium et de dibromo-1,4 benzène.

On a suivi le même mode opératoire que pour l'exemple 99 précèdent, mais en utilisant comme solvant le DMF au lieu du NMP.

Le bromo-4 phénylisocyanate isolé a été identifié par son point de fusion et ses spectres IR et RMN:
point de fusion: 41°C
spectre IR (dans $CH_2Cl_2$): $\nu$ NCO = 2240 cm$^{-1}$
Spectre RMN$^1$H (dans $CCl_4$): multiplet à 7,25 - 7,45 ppm (par rapport au TMS) correspondant aux 4 protons aromatiques.

**Exemple 102**

Préparation du méthoxy-4 phénylisocyanate à partir de cyanate de sodium et de bromo-4 anisole.
On a suivi le même mode opératoire que pour l'exemple 101.
Le méthoxy-4 phénylisocyanate isolé a été identifié par son indice de réfraction et ses spectres IR et RMN:

$$n_D^{20} = 1,5462$$

Spectre IR: $\nu$ NCO = 2255 cm$^{-1}$
Spectre RMN$^1$H (dans $CCl_4$): multiplet à 7,25 - 7,45 ppm (par rapport au TMS) correspondant aux 4 protons aromatiques -singulet à 3,7 ppm (par rapport au TMS) correspondant aux 3 protons du groupe méthoxy.

**Exemple 103**

Préparation d'isocyanate de butyle à partir de chlorure de butyle et de cyanate de sodium.
Dans un tube de Schlenk, muni d'un barreau aimanté et purgé à l'argon, on introduit successivement:

- 2,26 g (34,7 mmol) de cyanate de sodium sec,
- un mélange de 0,075 g (0,269 mmol) de bis (cyclooctadiène 1,5) nickel et de 0,107 g (0,269 mmol) de bis (diphénylphosphino)
-1,2 éthane (soit 0,269 mmol du complexe Ni (dppe)),
- 15 ml de DMF redistillé sur alumine,
- 2,49 g (26,9 mmol) de chlorure de butyle.

On agite et chauffe dans un bain d'huile thermostaté à 140°C le mélange pendant 24 h.
Après refroidissement, on filtre le mélange puis on distille à la pression atmosphérique le filtrat, à savoir tout d'abord le chlorure de butyle n'ayant pas réagi (76°C) puis l'isocyanate de butyle (115°C). L'isocyanate de butyle ainsi isolé a été identifié par spectrométrie IR et par CPG (comparaison avec un échantillon étalon).

**Exemple 104**

Préparation d'isocyanate de butyle à partir de chlorure de butyle et de cyanate de sodium.

Dans un réacteur de 100 cm$^3$, surmonté d'un réfrigérant et maintenu sous courant d'argon, on introduit successivement:

- 9,25 g (0,1 mole) de chlorure de butyle,
- 8,45 g (0,13 mole) de cyanate de sodium sec,
- 92,5 mg de Ni (dppe)$_2$ (1 % en poids par rapport au chlorure de butyle),
- 133,3 mg de dppe (diphénylphosphino)-1,2 éthane,
- 50 ml de DMAC.

On chauffe sous agitation pendant 4,5 heures à 150°C. Après refroidissement, on distille le mélange sous pression réduite pour séparer le mélange chlorure de butyle, isocyanate de butyle et DMAC du catalyseur.
Une distillation fractionnée du mélange sous pression atmosphérique a permis de récupérer de l'isocyanate de butyle, analysé et caractérisé par IR et CPG.
Le rendement obtenu en isocyanate de butyle est de 22 %.

**Tableau I . Préparation du phénylcarbamate d'éthyle à partir du bromobenzène**

| EX N° | Temp. C | Temps h | NCOM (mmol) | | PhBr (mmol) | Solvant (ml) | | Catalyseur (mmol) | | Ac. Lewis (mmol) | | EtOH (mmol) | Conv. % | Rdt. % | Observations |
|---|---|---|---|---|---|---|---|---|---|---|---|---|---|---|---|
| 1 | 155 | 27 | NCONa | 5,5 | 4,3 | DMF | 2,5 | Ni(dppe)$_2$ | 0,1 | – | | 4,6 | 68 | 27,5 | |
| 2 | 155 | 27 | NCONa | 5,3 | 4,2 | DMF | 2,4 | Ni(dppe)$_2$ | 0,093 | – | | 4,8 | 72 | 35 | 2x2,4mmol EtOH à 0 et 16h |
| 5 | 155 | 27 | NCONa | 8,1 | 4,1 | DMF | 3,7 | Ni(dppe)$_2$ | 0,098 | – | | 4,4 | 27 | 7 | |
| 6 | 155 | 27 | NCONa | 5,5 | 4,2 | DMAC | 3,2 | Ni(dppe)$_2$ | 0,1 | – | | 4,5 | 93 | 26,5 | |
| 7 | 155 | 27 | NCONa | 5,4 | 4,2 | DMAC | 3,0 | Ni(dppe)$_2$ | 0,1 | – | | 4,4 | 83 | 36 | 2x2,2mmol EtOH à 0 et 16h |
| 8 | 155 | 27 | NCONa | 5,5 | 4,2 | DMAC | 3,0 | Ni(dppe)$_2$ | 0,1 | SnCl$_2$ | 0,033 | 4,5 | 93 | 38 | 2x2,25mmol EtOH à 0 et 16h |
| 9 | 155 | 27 | NCONa | 5,5 | 4,3 | DMAC | 3,0 | Ni(dppe)$_2$ | 0,1 | SnCl$_2$ | 0,055 | 4,8 | 91 | 46 | 2x2,4mmol EtOH à 0 et 16h |
| 10 | 155 | 27 | NCONa | 5,4 | 4,2 | DMAC | 3,0 | Ni(dppe)$_2$ | 0,1 | SnCl$_2$ | 0,1 | 4,5 | 70 | 30 | 2x2,25 mmol EtOH à 0 et 16h |
| 11 | 155 | 27 | NCONa | 5,5 | 4,2 | DMAC | 3,0 | Ni(dppe)$_2$ | 0,1 | SnCl$_2$ | 0,5 | 4,6 | 80 | 13 | 2x2,23 mmol EtOH à 0 et 16h |
| 12 | 155 | 27 | NCONa | 5,5 | 4,2 | DMAC | 3,0 | Ni(dppe)$_2$ | 0,1 | SnCl$_2$ | 1,0 | 4,6 | 78 | 7 | 2x2,3mmol EtOH à 0 et 16h |
| 13 | 155 | 27 | NCONa | 5,4 | 4,3 | DMAC | 3,0 | Ni(dppe) | 0,1 | – | | 4,6 | 50 | 32 | |
| 14 | 155 | 27 | NCONa | 5,5 | 4,2 | DMAC | 3,0 | Ni(dppe) | 0,1 | SnCl$_2$ | 0,055 | 4,5 | 90 | 42 | |
| 15 | 155 | 27 | NCONa | 5,5 | 4,2 | DMAC | 3,0 | Ni(dppe) | 0,1 | SnCl$_2$ | 0,1 | 4,5 | 65 | 35,5 | |
| 16 | 155 | 27 | NCONa | 5,5 | 4,2 | DMAC | 3,0 | Ni(dppe) | 0,1 | SnCl$_2$ | 0,055 | 4,6 | 90 | 48 | 2x2,3mmol EtOH à 0 et 16h |

dppm = bis (diphénylphosphino) méthane
dppp = bis (diphénylphosphino) propane
ND = non déterminé

a) rendement en produit isolé

**Tableau I (suite) . Préparation du phénylcarbamate d'éthyle à partir du bromobenzène**

| EX N° | Temp. °C | Temps h | NCOM (mmol) | | PhBr (mmol) | Solvant (ml) | Catalyseur (mmol) | | Ac. Lewis (mmol) | | EtOH (mmol) | Conv. % | Rdt. % | Observations |
|---|---|---|---|---|---|---|---|---|---|---|---|---|---|---|
| 17 | 155 | 27 | NCONa | 5,5 | 4,3 | DMAC 3,0 | Ni(dppm)$_2$ | 0,1 | SnCl$_2$0,055 | 4,6 | | 83 | 23 | 2x2,3mmol EtOH à 0 et 16h |
| 18 | 155 | 27 | NCONa | 5,5 | 4,3 | DMAC 3,0 | Ni(dppb)$_2$ | 0,1 | SnCl$_2$0,055 | 4,6 | | 90 | 25[a] | 2x2,3mmol EtOH à 0 et 16h |
| 19 | 155 | 27 | NCONa | 5,5 | 4,3 | DMAC 3,0 | Ni(dppm) | 0,1 | SnCl$_2$0,055 | 4,6 | | 95 | 35 | 2x2,3mmolEtOH à 0 et 16h |
| 20 | 155 | 27 | NCONa | 5,5 | 4,3 | DMAC 3,0 | Ni(dppp) | 0,1 | SnCl$_2$0,055 | 4,6 | | 85 | 37[a] | 2x2,3mmol EtOH à 0 et 16h |
| 21 | 155 | 27 | NCONa | 5,5 | 4,3 | DMAC 3,0 | Ni(dppb) | 0,1 | SnCl$_2$0,055 | 4,6 | | 90 | 41[a] | 2x2,3mmol EtOH à 0 et 16h |
| 22 | 155 | 27 | NCONa | 5,5 | 4,3 | DMAC 3,0 | Ni(PBu$_3$)$_2$ | 0,1 | SnCl$_2$0,055 | 4,5 | | 90 | 30 | 2x2,25mmolEtOH à 0 et 16h |
| 23 | 155 | 27 | NCONa | 5,5 | 4,3 | DMAC 3,0 | Ni(PCy$_3$)$_2$ | 0,1 | SnCl$_2$0,055 | 4,5 | | 95 | 32 | 2x2,25mmol EtOH à 0 et 16h |
| 24 | 155 | 27 | NCONa | 5,4 | 4,3 | DMF 3,0 | Ni(dppe) | 0,1 | ZnCl$_2$0,055 | 4,6 | | 70 | 32 | |
| 25 | 155 | 27 | NCONa | 5,4 | 4,3 | DMF 3,0 | Ni (dppe) | 0,1 | ZnCl$_2$0,055 | 4,6 | | 75 | 32 | |
| 26 | 155 | 27 | NCONa | 5,4 | 4,3 | DMAC 3,0 | Ni (dppe) | 0,1 | ZnCl$_2$0,055 | 4,6 | | 75 | 32 | |
| 27 | 155 | 27 | NCONa | 5,4 | 4,3 | DMAC 3,0 | Ni (dppe) | 0,1 | ZnCl$_2$0,055 | 4,6 | | 80 | 37 | |
| 28 | 155 | 27 | NCONa | 5,5 | 4,3 | DMAC 3,0 | Ni(As Ph$_3$)$_2$ | 0,1 | SnCl$_2$0,055 | 4,7 | | 75 | 45[a] | 2x2,25mmol EtOH à 0 et 16h |
| 29 | 155 | 27 | NCONa | 5,5 | 4,3 | DMAC 3,0 | Ni(cod)$_2$ | 0,1 | SnCl$_2$0,055 | 4,7 | | 90 | 28[a] | 2x2,35mmol EtOH à 0 et 16h |
| 30 | 155 | 27 | (NOC$_2$)Ba | 3 | 4,3 | DMAC 3,0 | Ni(dppe)$_2$ | 0,1 | SnCl$_2$0,055 | 4,7 | | ND | ND | Formation de phénylcarbamate d'éthyle |
| 31 | 150 | 16 | NCOK | 6 | 4,5 | DMF 2,0 | Ni(dppe)(PPh$_3$) | 0,3 | – | 5,0 | | 40 | 10 | Formation de biphényle |
| 32 | 100 | 16 | NCOK | 6 | 4,5 | DMF 2,0 | Ni(PBu$_3$)$_2$ | 0,1 | – | 5,0 | | 10 | 2 | Formation de biphényle |

dppm = bis (diphénylphosphino) méthane
dppp = bis (diphénylphosphino) propane
ND = non déterminé

a) rendement en produit isolé

Tableau II  Préparation de la N-styryl N',N' diéthylurée et de la N-butyl N',N' diéthylurée.
Conditions expérimentales.

| Exemple | Temp. °C | Temps h | NCOM mmol | Dérivé halogéné mmol | Solvant ml | Catalyseur mmol | Ac.Lewis mmol | $Et_2NH$ mmol | Conv. % | Rdt % |
|---------|----------|---------|-----------|----------------------|------------|-----------------|---------------|---------------|---------|-------|
| 37 | 143 | 26 | NCONa 17,2 | Ph-Ch=CH-Br 14,3 | D.M.F. 10 | Ni(dppe)$_2$ 0,1 | - | 19 | 90 | 48 |
| 38 | 140 | 24 | NCONa 12,9 | $C_4H_9Cl$ 10 | D.M.F. 6 | Ni(dppe)2 0,1 | $SnCl_2$ 0,1 | 11 | 85 | 35 |

0 189 694

Tableau III . Préparation de différents arylcarbamates d'éthyle à partir d'halogénures aromatiques

| EX N° | Temp. °C | Temps h | NCOM (mmol) | Halogénure aromatique (mmol) | Solvant (ml) | Catalyseur (mmol) | Ac.Lewis (mmol) | EtOH (mmol) | Conv. % | Rdt. % | Observations |
|---|---|---|---|---|---|---|---|---|---|---|---|
| 33 | 155 | 26 | NCONa 364 | $C_6H_5Cl$ 282 | DMAC | Ni (dppe) 6,55 | $SnCl_2$ 3,6 | 310 | 90 | 37,5[b] | |
| 34 | 155 | 26 | NCONa 364 | $C_6H_5Cl$ 282 (bromo 4 | DMAC | Ni(dppe) 1,15 | $SnCl_2$ 3,6 | 310 | 82 | 26[b] | |
| 39 | 155 | 27 | NCONa 5,4 | p.BRC$_9$H$_{11}$cumène) | DMAC 3 | Ni(dppe) 0,1 | $SnCl_2$ 0,055 | 4,6 | 90 | 45[b] | |
| 40 | 155 | 27 | NCONa 5,4 | $C_6H_5Cl$ 4,2 | DMAC 3 | Ni(dppe) 0,1 | $SnCl_2$ 0,055 | 4,6 | 70 | 25[a] | |
| 41 | 155 | 27 | NCONa 5,4 | $C_6H_5Cl$ 4,2 | DMAC 3 | – | – | 4,6 | <1 | | Essai à blanc |
| 42 | 155 | 27 | NCONa' 5,4 | $C_6H_5Cl$ 4,2 | DMAC 3 | Ni(dppe) 0,1 | $SnCl_2$ 0,055 | 4,6 | >85 | 45[b] | 10% mol Bu$_4$N Br |
| 43 | 155 | 27 | NCONa 5,4 | $C_6H_5Cl$ 4,2 | DMAC 3 | Ni(dppe) 0,1 | $SnCl_2$ 0,055 | 4,6 | 85 | 27[b] | 5%mol Bu$_4$N Br |
| 44 | 155 | 27 | NCONa 5,4 | $C_6H_5Cl$ 4,2 | DMAC 3 | Ni(dppe) 0,1 | $SnCl_2$ 0,055 | 4,6 | 88 | 17[b] | 1 % mol Bu$_4$N Br |
| 45 | 155 | 27 | NCONa 5,4 | $C_6H_5Cl$ 4,2 | DMAC 3 | Ni(dppe) 0,1 | $SnCl_2$ 0,055 | 4,6 | 60 | 20[b] | 20%mol KBr |
| 46 | 155 | 27 | NCONa 5,4 | p-MeOC$_6$H$_4$Cl 4,2 | DMAC 3 | Ni(dppe) 0,1 | SnCl 0,055 | 4,6 | 50 | 14[b] | |
| 47 | 155 | 27 | NCONa 5,4 | p-MeOC$_6$H$_4$Cl 4,2 | DMAC 3 | Ni(dppe) 0,1 | $SnCl_2$ 0,055 | 4,6 | 65 | 32[b] | 10% mol Bu$_4$N Br |
| 48 | 155 | 27 | NCONa 5,4 | p-MeOC$_6$H$_4$Br 4,2 | DMAC 3 | Ni(dppe) 0,1 | $SnCl_2$ 0,055 | 4,6 | 60 | 47[b] | |
| 49 | 155 | 27 | NCONa 5,4 | p-MeOC$_6$H$_4$Br 4,2 | DMAC 3 | – | – | 4,6 | <1 | 0 | Essai à blanc |
| 50 | 155 | 27 | NCONa 5,4 | p-MeC$_6$H$_4$Br 4,2. | DMAC 3 | Ni(dppe) 0,1 | $SnCl_2$ 0,055 | 4,6 | 80 | 33[b] | |
| 51 | 155 | 27 | NCONa 5,4 | m-MeC$_6$H$_4$Br 4,2 | DMAC 3 | Ni(dppe) 0,1 | $SnCl_2$ 0,055 | 4,6 | 80 | 20[b] | |

a) détermine par CPG

b) isolé

Tableau III (suite) . Préparation de différents arylcarbamates d'éthyle à partir d'halogénures aromatiques

| EX N° | Temp. °C | Temps h | NCOM (mmol) | | Halogénure aromatique (mmol) | Solvant (ml) | | Catalyseur (mmol) | | Ac. Lewis (mmol) | | EtOH (mmol) | Conv. % | Rdt % | Observations |
|-------|----------|---------|-------|------|------------------|--------------|---|-----------|-----|---------|-------|---------|---------|---------|----------------|
| 52 | 155 | 27 | NCONa | 5,4 | p-ClC$_6$H$_4$Br 4;2 | DMAC | 3 | Ni(dppe) | 0,1 | SnCl$_2$ | 0,055 | 4,6 | >80 | 27[b] | monocarbamate |
| 53 | 155 | 27 | NCONa | 5,4 | o-ClC$_6$H$_4$Br 4,2 | DMAC | 3 | Ni(dppe) | 0,1 | SnCl$_2$ | 0,055 | 4,6 | 50 | 10[a] | monocarbamate |
| 54 | 155 | 27 | NCONa | 5,4 | p-FC$_6$H$_4$Br 4,2 | DMAC | 3 | Ni(dppe) | 0,1 | SnCl$_2$ | 0,055 | 4,6 | 70 | 18[b] | monocarbamate |
| 55 | 155 | 27 | NCONa | 5,4 | O$_2$N-C$_6$H$_4$Br 4;2 | DMAC | 3 | Ni(dppe) | 0,1 | SnCl$_2$ | 0,055 | 4,6 | 50 | 20[a] | |
| 56 | 155 | 27 | NCONa | 5,4 | F$_3$C-C$_6$H$_4$Br | DMAC | 3 | Ni(dppe) | 0,1 | SnCl$_2$ | 0,055 | 4,6 | 85 | 57[b] | |
| 57 | 155 | 27 | NCONa | 5,4 | p-C$_6$H$_4$Br$_2$ 4,2 | DMAC | 3 | Ni(dppe) | 0,1 | SnCl$_2$ | 0,055 | 4,6 | 70 | 32[b] | monocarbamate |
| 58 | 155 | 27 | NCONa | 5,4 | p-C$_2$H$_3$-C$_6$H$_4$Br 4,2 | DMAC | 3 | Ni(dppe) | 0,1 | SnCl$_2$ | 0,055 | 4,6 | – | 8[b] | |
| 59 | 155 | 27 | NCONa | 5,4 | 4,4'C$_{12}$H$_8$Br$_2$ 4,2 | DMAC | 3 | Ni(dppe) | 0,1 | SnCl$_2$ | 0,055 | 4,6 | 50 | 25[b] | monocarbamate |
| 60. | 155 | 27 | NCONa | 5,4 | m-C$_6$H$_4$Br$_2$ 4,2 | DMAC | 3 | Ni(dppe) | 0,1 | SnCl$_2$ | 0,055 | 4,6 | 50 | 22[b] | monocarbamate |
| 61 | 155 | 27 | NCONa | 5,4 | α-ClC$_{10}$H$_7$ 4,2 | DMAC | 3 | Ni(dppe) | 0,1 | SnCl$_2$ | 0,055 | 4,6 | 50 | 40[b] | |
| 62 | 155 | 27 | NCONa | 5,4 | α-ClC$_{10}$H$_7$ 4,2 | DMAC | 3 | Ni(dppe) | 0,1 | SnCl$_2$ | 0,055 | 4,6 | 75 | 53[b] | 10%mol Bu$_4$N Br |
| 63 | 155 | 27 | NCONa | 5,4 | α-BrC$_{10}$H$_7$ 4,2 | DMAC | 3 | Ni(dppe) | 0,1 | SnCl$_2$ | 0,055 | 4,6 | 90 | 52[b] | |
| 64 | 155 | 27 | NCONa | 5,4 | β-BrC$_{10}$H$_7$ 4,2 | DMAC | 3 | Ni(dppe) | 0,1 | SnCl$_2$ | 0,055 | 4,6 | 100 | 33[b] | |

a) déterminé par CPG

b) isolé

Tableau IV. Préparation du phényl-2 vinylcarbamate d'éthyle à partir du bromo-1 phényl-2 éthylène

| EX n° | Temp. °C. | Temps h | NCOM (mmol) | | PhCH= CHBr (mmol) | Solvant (ml) | | Catalyseur (mmol) | | Ac. Lewis (mmol) | | EtOH (mmol) | Conv. % | Rendement% (trans:cis) | Observations |
|---|---|---|---|---|---|---|---|---|---|---|---|---|---|---|---|
| 65 | 143 | 26 | NCONa | 17,2 | 14,5 | DMF | 10 | Ni(dppe)$_2$ | 0,1 | – | | 19 | 99 | 90 (91:9) | |
| 66 | 143 | 26 | NCONa | 17,1 | 14,5 | DMF | 10 | Ni(dppe)$_2$ | 0,1 | SnCl$_2$ | 0,055 | 19 | 85 | 16 (89:11) | |
| 67 | 143 | 26 | NCONa | 17,2 | 14,4 | DMF | 10 | Ni(dppe)$_2$ | 0,1 | – | | 19 | 100 | 76 (82:18) | 2x9,5mmol EtOH 0& 16h |
| 68 | 143 | 26 | NCONa | 17,2 | 14,5 | DMF | 10 | Ni(dppe)$_2$ | 0,1 | – | | 19 | 100 | 90 (91:9) | |
| 69 | 143 | 26 | NCONa | 17,2 | 14,5 | DMAC | 13,3 | Ni(dppe)$_2$ | 0,1 | SnCl$_2$ | 0,055 | 19 | 100 | 17,5(85:15) | |
| 70 | 143 | 26 | NCONa | 17,2 | 14,5 | DMAC | 13,3 | Ni(dppe)$_2$ | 0,1 | – | | 19 | 100 | 72 (88:12) | 2x9,5mmol EtOH 0& 16h |
| 71 | 143 | 26 | NCONa | 17,2 | 14,5 | DMAC | 13,3 | Ni(dppe) | 0,1 | – | | 19 | 90 | 35 (90:10) | |
| 72 | 155 | 27 | NCONa | 16,9 | 14,3 | DMF | 10 | Ni(PBu$_3$)$_4$ | 0,1 | SnCl$_2$ | 0,055 | 18,4 | 80 | 45 (88:12) | complexe formé in situ |
| 73 | 140 | 27 | NCONa | 16,9 | 14,3 | DMF | 10 | [CuI(PBu$_3$)]$_4$ | | – | | 15,7 | 80 | 0 | |

0189694

Tableau V. Préparation de différents vinylcarbamates d'éthyle à partir d'halogénures vinyliques

| Exemple n° | Temp. °C | Temps h | NCOM (mmol) | | Halogénure vinyl. (mmol) | | Solvant (ml) | | Catalyseur (mmol) | | EtOH (mmol) | Conv. % | Rdt. % | Observations |
|---|---|---|---|---|---|---|---|---|---|---|---|---|---|---|
| 75 | 143 | 26 | NCONa | 17,2 | PhCH=CHCl | 14,5 | DMF | 10 | Ni(dppe)$_2$ 0,1 | | 19 | 50 | 15 | |
| 76 | 143 | 26 | NCONa | 17,2 | PhCH=CHCl | 14,5 | DMF | 10 | Ni(dppe)$_2$0,1 | | 19 | 80 | 35 | 10% mol $|$Bu$_4$N$|$Br complexe formé in |
| 77 | 155 | 27 | NCONa | 16,9 | PhCH=CHCl | 14,3 | DMF | 10 | Ni(PBu$_3$)$_4$0,1 | | 18,4 | 80 | 45 | situ;+SnCl$_2$ 0,055mmol |
| 78 | 140 | 26 | NCONa | 16,9 | PhCH=CHCl | 14,3 | DMF | 10 | $|$CuI(PBu$_3$)$|$0,1 | | 15,7 | 80 | 0 | |
| 79 | 143 | 26 | NCONa | 17, 2 | 3,4(MeO)$_2$C$_6$H$_3$-C$_2$H$_2$Br 14,5 | | DMF | 10 | Ni(dppe)$_2$0,1 | | 19 | 100 | 50 | |
| 80 | 143 | 26 | NCONa | 17,2 | EtOCH=CHBr | 14,5· | DMF | 10 | Ni(dppe)$_2$0,1 | | 19 | 70 | 11 | en tube de Schlenk |
| 81 | 143 | 26 | NCONa | 17,2 | MeCH=CHBr | 14,5 | DMF | 10 | Ni(dppe)$_2$0,1 | | 19 | 85 | 26 | en tube de Schlenk |
| 82 | 143 | 26 | NCONa | 17,2 | CH$_2$=CHBr | 14,5. | DMF | 10 | Ni(dppe)$_2$0,1 | | 19 | – | 15 | en autoclave |

0 189 694

**Tableau VI. Préparation de carbamates d'éthyle à partir d'hétérocycles halogénés**

| Exemple n° | Temp. °C | Temps h | NCOM (mmol) | | Halogénure hétéro (mmol) | | Solvant (ml) | Catalyseur (mmol) | EtOH mmol) | Conv. % | Rdt. % | Observations |
|---|---|---|---|---|---|---|---|---|---|---|---|---|
| 83 | 140 | 27 | NCONa | 5,5 | $2\text{-}BrC_5H_4N$ | 4,3 | DMAC3 | Ni(dppe)0,1 | 4,7 | 35 | 15 | - |
| 84 | 140 | 27 | NCONa | 5,5 | $2\text{-}BrC_5H_4N$ | 4,3 | DMAC 3 | Ni(dppe)0,1 | 4,7 | 25 | 7 | $SnCl_2$ 0,055mmol |
| 85 | 140 | 27 | NCONa | 5,4 | $3\text{-}BrC_5H_4N$ | 4,3 | DMAC 3 | Ni(dppe)0,1 | 4,6 | 60 | 31 | $SnCl_2$ 0,055mmol. |
| 86 | 140 | 27 | NCONa | 5,5 | $4\text{-}BrC_5H_4NH^+Cl\text{-}4,3$ | | DMAC 3 | Ni(dppe)0,1 | 4,7 | - | ≈4 | $SnCl_2$ 0,055mmol |
| 87 | 140 | 27 | NCONa | 5,4 | $4\text{-}BrC_9H_7N$ | 4,3 | DMAC 3 | Ni(dppe)0,1 | 4,7 | 70 | 25 | $SnCl_2$ 0,055mmol |
| 88 | 140 | 27 | NCONa | 5,4 | $3\text{-}BrC_4H_3O$ | 4,3 | DMAC 3 | Ni(dppe)0,1 | 4,7 | 90 | 10 | $SnCl_2$ 0,055mmol |

0 189 694

Tableau VII. Préparation d'allyl-, benzyl- et alkylcarbamates d'éthyle
à partir des halogénures organiques correspondants

| Exemple n° | Temp. °C | Temps h | NCOM (mmol) | Halogénure orga. (mmol) | Solvant ml | Catalyseur (mmol) | EtOH (mmol) | Conv. % | Rdt. % | Observations |
|---|---|---|---|---|---|---|---|---|---|---|
| 89 | 140 | 24 | NCONa 12, | Me-2 $C_3H_4Cl$ 10 | DMF 6 | Ni (dppe)0,1 | 11 | 90 | 41 | $SnCl_2$ 0,1mmol |
| 90 | 140 | 24 | NCONa 12, | Me-2 $C_3H_4Cl$ 10 | DMF 6 | – | 11 | 90 | 33 | essai à blanc |
| 91 | 140 | 24 | NCONa 12, | $PhC_3H_4Br$ 10 | DMF 6 | Ni(dppe) 0,1 | 11 | .85 | 30 | $SnCl_2$ 0,1mmol |
| 92 | 140 | 24 | NCONa 12, | $PhC_3H_4Br$ 10 | DMF 6 | –. | 11 | 80 | 20 | essai à blanc |
| 93 | 155 | 24 | NCONa 12, | $PhCH_2Cl$ 10 | DMF 6 | Ni (dppe)0,1 | 11 | 90 | 73 | – |
| 94 | 155 | 24 | NCONa 12, | $PhCH_2Cl$ 10 | DMF 6 | Ni (dppe) | 11 | 90 | 60 | SnCl 0,1 mmol |
| 95 | 140 | 24 | NCONa 12, | PhCH Cl 10 | DMF 6 | – | 11 | ·90 | 43 | essai à blanc |
| 96 | 140 | 24 | NCONa 12, | $C_4H_9Cl$ 10 | DMF 6 | Ni(dppe) 0,1 | 11 | 95 | 70 | – |
| 97 | 140 | 24 | NCONa 12,9 | $C_4H_9Cl$ 10 | DMF 6 | Ni(dppe) 0,1 | 11 | 90 | 62 | $SnCl_2$0,1 mmol |
| 98 | 140 | 24 | NCONa 12,9 | $C_4H_9Cl$ 10 | DMF 6 | – | 11 | 90 | 35 | essai à blanc |

0189 694

## Revendications

1. Procédé de synthèse d'isocyanates par réaction, en milieu organique, d'un halogénure organique avec un cyanate métallique caractérisé en ce que la réaction précitée a lieu en présence d'un catalyseur constitué par un complexe du nickel avec au moins un ligand organique, complexe dans lequel le nickel est au degré d'oxydation zéro.

2. Procédé de synthèse de dérivés d'isocyanates de formule générale RNHCOZ dans laquelle R représente un groupement organique et Z un groupement choisi dans le groupe constitué par les groupements alcoxy et aminés caractérisé en ce qu'on met en oeuvre le procédé selon la revendication 1 puis en ce qu'on fait réagir l'isocyanate avec un composé de formule ZH, Z ayant la signification précitée.

3. Procédé de synthèse de carbamates selon la revendication 2 caratérisé en ce qu'on fait réagir l'isocyanate avec un composé hydroxylé.

4. Procédé de synthèse d'urées selon la revendication 2 caractérisé en ce qu'on fait réagir l'isocyanate avec une amine primaire ou secondaire.

5. Procédé de synthèse selon l'une quelconque des revendications 2 à 4, caractérisé en ce que le rapport molaire composé ZH/halogénure organique est compris entre 1,15 et 1,35.

6. Procédé selon l'une quelconque des revendications précédentes caractérisé en ce que l'halogénure organique est choisi dans le groupe constitué par:
- les halogénures vinyliques, acycliques ou cyclitues, éventuellement substitués par au moins un groupement alkyle ou aryle;
- les halogénures aromatiques simples ou condensés, éventuellement substitués par au moins un groupement alkyle, vinyle, aryle, halogène, alcoxy;
- les composés hétérocycliques halogénés,
- les halogénures allyliques, éventuellement substitués par au moins un groupement alkyla ou aryle;
- les halogénures aliphatiques linéaires ou ramifiés ou cycliques;
- les halogénures benzyliques, éventuallement substitués par au moins un groupement alkyle ou aryle.

7. Procédé salon l'une quelconque des revendications précédentes caractérisé an ca que l'halogénure organique est choisi dans le groupe constitué par:
-les halogénures vinyliques, acycliques ou cycliques, éventuellement substitués par au moins un groupement alkyle ou aryle,
-les halogénures aromatiques simples ou condensés, éventuellement substitués par au moins un groupement alkyle, vinyle, aryle halogène, ou alcoxy.

8. Procédé selon l'une quelconque des revendications précédentes caractérisé en ce que l'halogénure organique est un chlorure organique et en ce qu'on ajoute au milieu réactionnel un bromure ou iodure alcalin ou d'onium.

9. Procédé selon la revendication 8 caractérisé en ce que la fraction molaire du bromure ou iodure alcalin ou d'onium ajouté par rapport au chlorure organique est de l'ordre de 10 %.

10. Procédé selon l'une quelconque des revendications précédentes caractérisé en ce que le cyanate métallique est le cyanate de sodium.

11. Procédé selon l'une quelconque des revendications précédentes caractérisé en ce que le ou les ligands organiques sont choisis dans le groupe constitué par les arsines tertiaires, les phosphines basiques et les phosphines bidentates.

12. Procédé selon la revendication il caractérisé en ce que la phosphine basique est choisie dans le groupe constitué par la triphénylphosphine, la tributylphosphine at la tricyclohexylphosphine.

13. Procédé selon la revendication 11 caractérisé an ce que la phosphine bidentate est un bis (diphénylphosphino)polyméthylène.

14. Procédé selon la ravendication 13 caractérisé an ce que le bis (diphénylphosphino)polyméthylène est le bis (diphénylphosphino)éthane ou le bis (diphénylphosphino)butane.

15. Procédé selon la revendication 11 caractérisé en ce que l'arsine tertiaire est la triphénylarsine.

16. Procédé selon l'une quelconque des revendications précédentes caractérisé en ce que la réaction a également lieu en présence d'un acide de Lewis.

17. Procédé selon l'une quelconque des revendications précédentes caractérisé en ce que le milieu organique contient un solvant aprotique dipolaire.

18. Procédé selon la revendication 17 caractérisé en ce que le milieu organique contient également un solvant hydrocarboné.

19. Procédé selon l'une quelconque des revendications précédentes caractérisé en ce que, avant l'addition du catalyseur, on effectue une distillation azéotropique de l'eau du mélange réactionnel.

## Patentansprüche

1. Verfahren zur Herstellung von Isocyanaten durch Umsetzung im organischen Medium eines organischen Halogenids mit einem Metallcyanat,
dadurch gekennzeichnet, daß die Umsetzung in Gegenwart eines Katalysators, der ein Nickelkomplex mit

mindestens einem organischen Ligan den ist, wobei das Nickel die Oxidationsstufe Null hat, durchgeführt wird.

2. Verfahren zur Herstellung von Isocyanatderivaten der allgemeinen Formel RNHCOZ,

in der R ein organischer Rest ist und Z unter Alkoxy- und Aminogruppen gewählt wird,

dadurch gekennzeichnet, daß nach Durchführung des Verfahrens nach Anspruch 1 das Isocyanat mit einer Verbindung der Formel ZH umgesetzt wird, wobei Z die oben angegebene Bedeutung hat.

3. Verfahren nach Anspruch 2 zur Herstellung von Carbamaten, dadurch gekennzeichnet, daß das Isocyanat mit einer Hydroxylverbindung umgesetzt wird.

4. Verfahren nach Anspruch 2 zur Herstellung von Harnstoffen, dadurch gekennzeichnet, daß das Isocyanat mit einem primären oder sekundären Amin umgesetzt wird.

5. Herstellungsverfahren nach einem der Ansprüche 2 bis 4, dadurch gekennzeichnet, daß ein molares Verhältnis von Verbindung der Formel ZH zu organischem Halogenid von 1,15 bis 1,35 eingesetzt wird.

6. Verfahren nach einem der vorstehenden Ansprüche, dadurch gekennzeichnet, daß das organische Halogenid unter folgenden Verbindungen gewählt wird:

- acyclischen oder cyclischen Vinylhalogeniden, die gegebenenfalls mit mindestens einer Alkyl- oder Arylgruppe substituiert sind;

-einfachen oder kondensierten aromatischen Halogeniden, die gegebenenfalls mit mindestens einer Alkyl-, Vinyl-, Aryl- oder Alkoxygruppe oder einem Halogenatom substituiert sind;

- halogenierten heterocyclischen Verbindungen,

- Allylhalogeniden, die gegebenenfalls mit mindestens einer Alkyl- oder Arylgruppe substituiert sind;

- geradkettigen, verzweigten oder cyclischen aliphatischen Halogeniden;

- Benzylhalogeniden, die gegebenenfalls mit mindestens einer Alkyl- oder Arylgruppe substituiert sind.

7. Verfahren nach einem der Vorstehenden Ansprüche, dadurch gekennzeichnet, daß das organische Halogenid unter folgenden Verbindungen gewählt wird:

- acyclischen oder cyclischen Vinylhalogeniden, die gegebenenfalls mit mindestens einer Alkyl- oder Arylgruppe substituiert sind,

-einfachen oder kondensierten aromatischen Halogeniden, die gegebenenfalls mit mindestens einer Alkyl-, Vinyl-, Aryl- oder Alkoxygruppe oder einem Halogenatom substituiert sind.

8. Verfahren nach einem der Vorstehenden Ansprüche, dadurch gekennzeichnet, daß als organisches Halogenid ein organisches Chlorid verwendet wird und daß dem Reaktionsmedium ein Alkali- oder Oniumbromid oder -jodid zugegeben wird.

9. Verfahren nach Anspruch 8,

dadurch gekennzeichnet, daß das Alkali- oder Oniumbromid oder -jodid in einem molaren Verhältnis zum organischen Chlorid in der Größenordnung von 10 % zugegeben wird.

10. Verfahren nach einem der vorstehenden Ansprüche, dadurch gekennzeichnet, daß als Metallcyanat Natriumcyanat verwendet wird.

11. Verfahren nach einem der vorstehenden Ansprüche, dadurch gekennzeichnet, daß der organische Ligand oder die organischen Liganden unter tertiären Arsinen, basischen Phosphinen und zweizähnigen Phosphinen gewählt wird (werden).

12. Verfahren nach Anspruch 11, dadurch gekennzeichnet, daß das basische Phosphin unter Triphenylphosphin, Tributylphosphin und Tricyclohexylphosphin gewählt wird.

13. Verfahren nach Anspruch 11, dadurch gekennzeichnet, daß als zweizähniges Phosphin ein Bis (diphenylphosphino)polymethylen verwendet wird.

14. Verfahren nach Anspruch 13, dadurch gekennzeichnet, daß als Bis (diphenylphosphino)polymethylen Bis (diphenylphosphino)ethan oder Bis (diphenylphosphino)butan verwendet wird.

15. Verfahren nach Anspruch 11, dadurch gekennzeichnet, daß als tertiäres Arsin Triphenylarsen verwendet wird.

16. Verfahren nach einem der vorstehenden Ansprüche, dadurch gekennzeichnet, daß die Umsetzung auch in Gegenwart einer Lewissäure durchgeführt wird.

17. Verfahren nach einem der vorstehenden Ansprüche, dadurch gekennzeichnet, daß ein organisches Medium, das ein aprotisches dipolares Lösungsmittel enthält, verwendet wird.

18. Verfahren nach Anspruch 17, dadurch gekennzeichnet, daß ein organisches Medium verwendet wird, das auch ein Kohlenwasserstoff-Lösungsmittel enthält.

19. Verfahren nach einem der vorstehenden Ansprüche, dadurch gekennzeichnet, daß vor der Zugabe des Katalysators das Wasser vom Reaktionsmedium azeotrop abdestilliert wird.

## Claims

1. Process for the synthesis of isocyanates by reacting an organic halide with a metal cyanate in an organic medium characterized in that the abovementioned reaction takes place in the presence of a catalyst consisting of a complex of nickel with at least one organic ligand, in which complex the nickel is in an oxidation level of zero.

2. Process for the synthesis of isocyanate derivatives of general formula RNHCOZ, in which R represents an organic group and Z a group chosen from the category consisting of alkoxy groups and amine groups, characterized in that the process according to claim 1 is employed and that thereafter the isocyanate is reacted with a compound of formula ZH, 2 having the abovementioned meaning.

3. Process for the synthesis of carbamates according to claim 2, characterized in that the isocyanate is reacted with a hydroxyl compound.

4. Process for the synthesis of ureas according to claim 2, characterized in that the isocyanate is reacted with a primary or secondary amine.

5. Process of synthesis according to any one of claims 2 to 4, characterized in that the molar ratio of compound ZH to organic halide is between 1.15 and 1.35.

6. Process according to any one of the preceding claims, characterized in that the organic halide is chosen from the group consisting of:

vinyl, acyclic or cyclic halides, optionally substituted by at least one alkyl or aryl group,

simple or condensed aromatic halides, optionally substituted by at least one alkyl, vinyl, aryl, halogen or alkoxy group,

halogenated heterocyclic compounds,

allyl halides optionally substituted by at least one alkyl or aryl group,

linear or branched or cyclic aliphatic halides and benzyl halides optionally substituted by at least one alkyl or aryl group.

7. Process according to any one of the preceding claims, characterized in that the organic halide is chosen from the group consisting of:

vinyl, acyclic or cyclic halides, optionally substituted by at least one alkyl or aryl group and

simple or condensed aromatic halides optionally substituted by at least one alkyl, vinyl, aryl, halogen or alkoxy group.

8. Process according to any one of the preceding claims, characterized in that the organic halide is an organic chloride and in that an alkali metal bromide or iodide or onium bromide or iodide is added to the reaction mixture.

9. Process according to claim 8, characterized in that the mole fraction of alkali metal bromide or iodide or onium bromide or iodide is of the order of 10 % relative to the organic chloride.

10. Process according to any one of the preceding claims, characterized in that the metal cyanate is sodium cyanate.

11. Process according to any one of the preceding claims, characterized in that the organic ligand or ligands is or are chosen from a group consisting of tertiary arsines, basic phosphines and bidentate phosphines.

12. Process according to claim 11, characterized in that the basic phosphine is chosen from a group consisting of triphenylphosphine, tributylphosphine and tricyclohexylphosphine.

13. Process according to claim 11, characterized in that the bidentate phosphine is a bis(diphenylphosphino)polymethylene.

14. Process according to claim 13, characterized in that the bis(diphenylphosphino)polymethylene is bis (diphenylphosphino)ethane or bis(diphenylphosphino)butane.

15. Process according to claim 11, characterized in that the tertiary arsine is triphenylarsine.

16. Process according to any one of the preceding claims, characterized in that the reaction is also carried out in the presence of a Lewis acid.

17. Process according to any one of the preceding claims, characterized in that the organic medium contains a dipolar aprotic solvent.

18. Process according to claim 17, characterized in that the organic medium also contains a hydrocarbon solvent.

19. Process according to any one of the preceding claims, characterized in that before addition of the catalyst, the water is distilled azeotropically from the reaction mixture.